Europäisches Patentamt

European Patent Office

Office européen des brevets

(19)

(11) EP 0 591 534 B1

(12) EUROPEAN PATENT SPECIFICATION

(45) Date of publication and mention
of the grant of the patent:
**06.11.1996 Bulletin 1996/45**

(21) Application number: **92911803.2**

(22) Date of filing: **12.06.1992**

(51) Int Cl.6: **C07G 17/00**, C12P 1/04
// A61K35/74,(C12P1/04,
C12R1:38)

(86) International application number:
**PCT/JP92/00754**

(87) International publication number:
**WO 92/22562 (23.12.1992 Gazette 1992/32)**

(54) **SUBSTANCES WB2663, PRODUCTION THEREOF AND USE**

SUBSTANZEN WB2663, IHRE HERSTELLUNG UND VERWENDUNG

SUBSTANCES WB2663, LEUR PRODUCTION ET LEUR UTILISATION

(84) Designated Contracting States:
**CH DE FR GB IT LI**

(30) Priority: **14.06.1991 JP 169104/91**

(43) Date of publication of application:
**13.04.1994 Bulletin 1994/15**

(73) Proprietor: **FUJISAWA PHARMACEUTICAL CO.,
LTD.
Osaka-shi Osaka 541 (JP)**

(72) Inventors:
• **NAKAJIMA, Hidenori
  Ibaraki-ken 305 (JP)**
• **HORI, Yasuhiro
  Tsukuba-gun Ibaraki-ken 300-24 (JP)**
• **GOTO, Toshio
  Ibaraki-ken 305 (JP)**
• **TAKASE, Shigehiro
  Ibaraki-ken 315 (JP)**
• **VERHAEGHE, Koen
  Tsukuba-shi Ibaraki-ken 305 (JP)**

• **TERANO, Hiroshi
  Ibaraki-ken 300 (JP)**
• **OKUHARA, Masakuni
  Ibaraki-ken 305 (JP)**

(74) Representative: **Tiedtke, Harro, Dipl.-Ing.
Patentanwaltsbüro
Tiedtke-Bühling-Kinne & Partner
Bavariaring 4
80336 München (DE)**

(56) References cited:
• **CHEMICAL ABSTRACTS, vol. 106, no. 5, 2
  February 1987, Columbus, Ohio, US; abstract
  no. 31372j, OGAWARA, HIROSHI ET AL.
  'Manufacture of antitumor agent YM-3229G-A by
  Pseudomonas' page 407 ;column R ;**
• **The Journal of Antibiotics, Vol. 43, 1990, J.
  SHOJI et al., "Isolation of Cepafungins I, II and III
  from Pseudomonas species", p. 783-787.**
• **The Journal of Antibiotics, Vol. 41, 1988, J.
  SHOJI et al., "Isolation of a new phenazine
  antibiotic DOB-41 Pseudomonas species", p.
  589-594.**

**Description**

Field of the Invention

The present invention relates to WB2663 substances, a method for their production, and use thereof. WB2663 substances are novel substances hitherto unknown, which were separated and collected from a culture of Pseudomonas cells and which exhibit an excellent antitumor effect and are particularly useful as prophylactic and/or treatment agent for various types of tumors.

Description of the Prior Art

In the past, various types of antitumor agents have been developed from naturally occurring substances by methods of extraction, fermentation and chemical synthesis, etc., but as few of these exhibit a strong antitumor effect without harmful side effects, it has been earnestly desired to develop more excellent antitumor agents.

Problem to be solved by the Invention

The present invention was undertaken in light of the above mentioned state of the current technology, with the object of developing an excellent antitumor agent which is highly safe and has a wide-ranging and powerful antitumor effect.

Means to solve the Problem

The present invention was undertaken in order to achieve the above mentioned object.

We the present inventors focused on natural substances for the sake of safety, finally concentrating our attention on microbial fermentates, and upon investigation of various microorganisms we discovered that microorganism No. 2663 strain newly separated from soil taken in Mie Prefecture accumulates the substance of interest in a culture solution. Furthermore, upon more detailed research regarding the physicochemical properties of the substance, we discovered that it is not a single substance but rather a mixture of a number of different substances, and we succeeded in isolating 3 of these substances. These substances were confirmed to be novel substances unknown to the prior art, and were collectively named WB2663 substances, and respectively WB2663A substance, WB2663B substance and WB2663C substance. Further research resulted in the establishment of a method for their industrial production, and the present invention was thus completed.

Brief Description of the Drawings

Fig. 1 is a drawing showing a $^{13}$C nuclear magnetic resonance spectrum of WB2663A substance.
Fig. 2 is a drawing showing a $^{1}$H nuclear magnetic resonance spectrum of WB2663A substance.
Fig. 3 is a drawing showing a $^{13}$C nuclear magnetic resonance spectrum of WB2663B substance.
Fig. 4 is a drawing showing a $^{1}$H nuclear magnetic resonance spectrum of WB2663B substance.
Fig. 5 is a drawing showing a $^{13}$C nuclear magnetic resonance spectrum of WB2663C substance.
Fig. 6 is a drawing showing a $^{1}$H nuclear magnetic resonance spectrum of WB2663C substance.
WB2663A substance, WB2663B substance and WB2663C substance according to the present invention each possess the physicochemical properties listed below.

Physicochemical properties of WB2663A substance

(1) Color and form of substance
    White needle-shaped crystals
(2) Melting point
    102-104°C
(3) Specific rotation
    $[\alpha]_D^{23}$: -36° (c=0.5, $CH_2Cl_2$)
(4) Molecular formula
    $C_{27}H_{42}ClNO_8$
(5) Elemental analysis

Calculated for $C_{27}H_{42}ClNO_8$ (%):

C, 59.60; H, 7.78; N, 2.57; Cl, 6.52

Found (%):

C, 60.11; H, 8.10; N, 2.44; Cl, 6.86

(6) Solubility in solvents
     Readily soluble: dichloromethane, chloroform, acetone, ethyl acetate
     Insoluble: hexane, water

(7) Color reaction

Positive:     iodine vapor reaction, cerium sulfate reaction, potassium permanganate reaction
Negative:     ferric chloride reaction, Ehrlich reaction, ninhydrin reaction

(8) Thin-layer chromatography

| Stationary phase | Developing solvent | Rf value |
|---|---|---|
| Silica gel (Silica Gel 60 $F_{254}$ (product of E. Merck Co.) | dichloromethane:methanol (20:1) | 0.47 |
|  | dichloromethane:acetone (2:3) | 0.74 |

(9) Infrared absorption spectrum
     $\nu_{max}^{KBr}$ 3400, 2980, 2940, 1735, 1665, 1630, 1530, 1370, 1240, 1055 cm$^{-1}$

(10) High performance liquid chromatography

Column: YMC AM-303 [S-5, 120A, ODS, 4.6 mmID x 250 mm; product of Yamamura Chemical Laboratories, Ltd.]
Detection: 210 nm
Developing solvent: 70% aqueous solution of methanol
Flow rate: 1 ml/min
Retention time (RT): 7.9 min

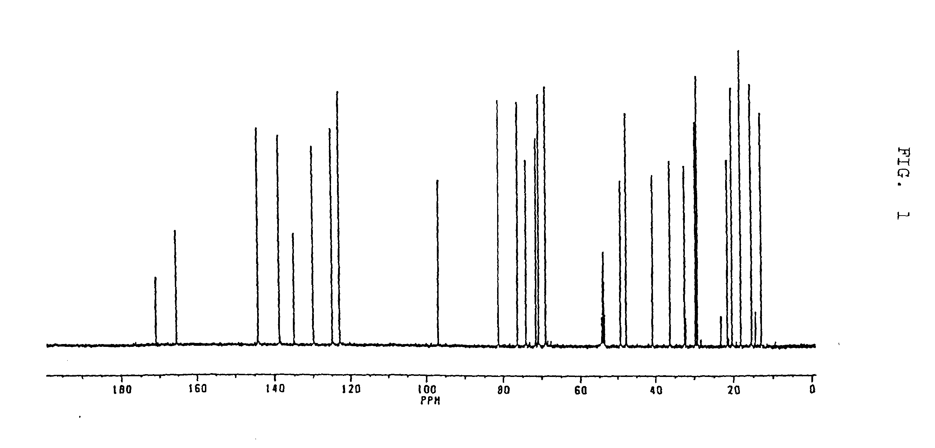

(11) $^{13}$C nuclear magnetic resonance spectrum
     (100 MHz, $CD_2Cl_2$)
     Chart shown in Fig. 1

$\delta_c$:    170.8(s), 165.4(s), 144.1(d), 138.7(d), 134.9(s), 129.9(d), 124.8(d), 122.7(d), 96.8(s), 81.3(d), 76.3(d), 74.0 (s), 71.4(d), 70.7(d), 68.9(d), 49.2(t), 47.7(d), 40.7(t), 36.2(t), 32.4(t), 29.6(d), 29.1(q), 21.4(q), 20.2(q), 18.0 (q), 15.3(q), 12.8(q).

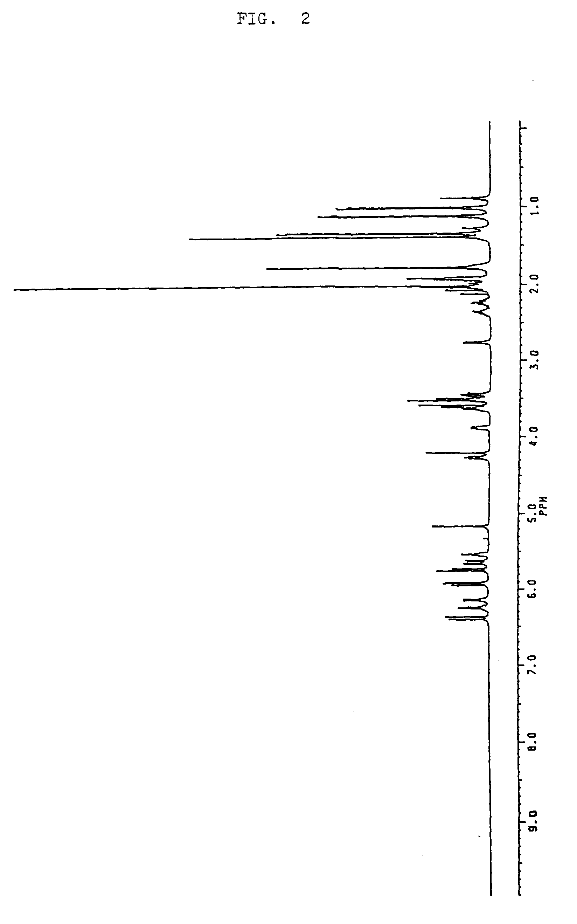

(12) $^1$H nuclear magnetic resonance spectrum
     (400 MHz, $CD_2Cl_2$)
     Chart shown in Fig. 2

$\delta_H$:    6.37(1H, d, J=16Hz), 6.24 (1H, m), 6.13(1H, d, J=9Hz, exchangeable), 5.91(1H, dd, J=11.5, 8Hz), 5.73(1H, dd, J=11.5, 1Hz), 5.64(1H, dd, J=16, 7Hz), 5.53(1H, m), 5.16(1H, s, exchangeable), 4.27(1H, dd, J=9.5, 7Hz), 4.20(1H, s, exchangeable), 3.88(1H, m), 3.63(1H, m), 3.59(1H, d, J=11Hz), 3.51(1H, d, J=11Hz), 3.50 (1H, m), 3.44(1H, dd, J=9.5, 7.5Hz), 2.76(1H, d, J=7.5Hz, exchangeable), 2.35(1H, m), 2.22(1H, m), 2.05 (1H, d, J=14Hz), 2.01(3H, s), 1.92(1H, d, J=14Hz), 1.91(2H, m), 1.78 (3H, br s), 1.75(1H, m), 1.38(3H, s), 1.34(3H, d, J=6.5Hz), 1.11(3H, d, J=6.5Hz), 1.00(3H, d, J=7.5Hz).

(13) Molecular formula and molecular weight
$C_{27}H_{42}ClNO_8$
    FAB-MS m/z 566 $(M+Na)^+$
(14) Acidity/basicity classification of substance
    Neutral substance
(15) Ultraviolet absorption spectrum
    $\lambda_{max}^{acetonitrile}$ nm ($\in$): 234 (38,000)

Physicochemical properties of WB2663B substance

(1) Color and form of substance
    White powder
(2) Melting point
    65-70°C
(3) Specific rotation
    $[\alpha]_D^{23}$: -12° (c=0.5, $CH_2Cl_2$)
(4) Molecular formula and molecular weight

    FAB-MS m/z 508 $(M+H)^+$
    HRFAB-MS m/z 508.2929
    (Calculated for $C_{27}H_{41}NO_8$ + H : 508.2910)

(5) Solubility in solvents

    Readily soluble: ethyl acetate, dichloromethane, acetonitrile, chloroform
    Hardly soluble: water
    Insoluble: hexane

(6) Color reaction

Positive:    iodine vapor reaction, cerium sulfate reaction, potassium permanganate reaction
Negative:    ferric chloride reaction, ninhydrin reaction, Ehrlich reaction

(7) Thin-layer chromatography

| Stationary phase | Developing solvent | Rf value |
|---|---|---|
| Silica Gel 60 $F_{254}$ (product of E. Merck Co.) | dichloromethane:acetone (2:3) | 0.50 |
| RP-18W $F_{254}$S (product of E. Merck Co.) | acetonitrile:water (60:40) | 0.18 |

(8) High performance liquid chromatography

    Column: YMC AM-303 [S-5, 120A, ODS, 4.6 mmID x 250 mm; product of Yamamura Chemical Laboratories, Ltd.]
    Detection: 210 nm
    Developing solvent: 40% aqueous acetonitrile
    Flow rate: 1 ml/min
    Retention time (RT): 12.2 min

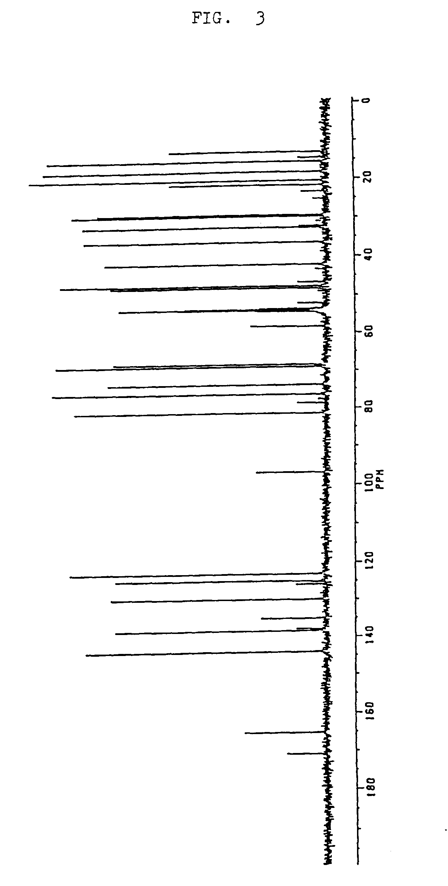

(9) $^{13}C$ nuclear magnetic resonance spectrum
    (100 MHz, $CD_2Cl_2$)
    Chart shown in Fig. 3

$\delta_c$:    170.7(s), 165.0(s), 143.9(d), 138.3(d), 134.9(s), 129.8(d), 124.8(d), 122.9(d), 96.7(s), 81.2(d), 76.3(d), 73.8(d), 68.9(d), 68.2(d), 58.2(s), 48.1(t), 47.5(d), 41.9(t), 36.3(t), 32.4(t), 29.6(d), 29.2(q), 21.4(q), 20.2(q), 18.0(q), 15.3(q), 12.8(q).

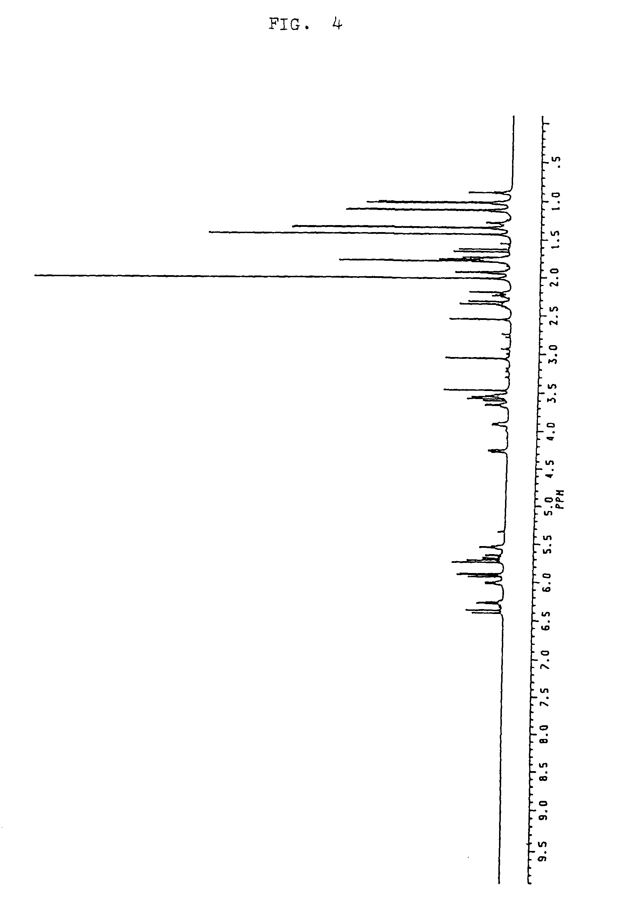

(10) $^1$H nuclear magnetic resonance spectrum
(400 MHz, $CD_2Cl_2$)
Chart shown in Fig. 4
The significant signals were as follows:

$\delta_H$: 6.38(1H, d, J=16Hz), 6.26(1H, m), 6.00(1H, d, J=9Hz, exchangeable), 5.89(1H, dd, J=11.5 and 8Hz), 5.72 (1H, dd, J=11.5 and 1Hz), 5.66(1H, dd, J=16 and 7Hz), 5.53(1H, m), 4.26(1H, m), 3.89(1H, m), 3.68-3.50 (3H, m), 3.46(1H, s, exchangeable), 3.05(1H, d, J=4.5Hz), 2.54(1H, d, J=4.5Hz), 2.36(1H, m), 2.32(1H, d, J=14Hz), 2.23(1H, m), 2.01(3H, s), 1.92(2H, m), 1.78(3H, br s), 1.75(1H, m), 1.64(1H, d, J=14Hz), 1.42(3H, s), 1.33(3H, d, J=6.5Hz), 1.11(3H, d, J=6.5Hz), 1.01(3H, d, J=7.5Hz).

(11) Acidity/basicity classification of substance
Neutral substance

(12) Ultraviolet absorption spectrum
$\lambda_{max}^{acetonitrile}$ nm(E): 235 (30,500)

(13) Infrared absorption spectrum
$\nu_{max}^{KBr}$: 3400, 2980, 2930, 1735, 1665, 1640, 1630, 1530, 1370, 1250, 1050, 970 cm$^{-1}$

Physicochemical properties of WB2663C substance

(1) Color and form of substance
White powder

(2) Melting point
86-91°C

(3) Specific rotation
$[\alpha]_D^{23}$: -9° (c=0.5, $CH_2Cl_2$)

(4) Molecular formula and molecular weight

FAB-MS m/z 524 (M+H)$^+$
HRFAB-MS m/z 524.2849
(Calculated for $C_{27}H_{41}NO_9$ + H : 524.2860)

(5) Solubility in solvents

Readily soluble: acetonitrile, chloroform, dichloromethane
Hardly soluble: water
Insoluble: n-hexane, cyclohexane, carbon tetrachloride

(6) Color reaction

Positive:     cerium sulfate reaction, potassium permanganate reaction
Weakly positive: iodine vapor reaction
Negative:     ferric chloride reaction, ninhydrin reaction, Ehrlich reaction

(7) Thin-layer chromatography

| Stationary phase | Developing solvent | Rf value |
|---|---|---|
| Silica Gel 60 $F_{254}$ (product of E. Merck Co.) | acetone | 0.44 |
| | dichloromethane:acetone (2:3) | 0.08 |
| RP-18 WF$_{254}$S (product of E. Merck Co.) | acetonitrile:water (60:40) | 0.81 |

(8) High performance liquid chromatography

Column: YMC AM-303 [S-5, 120A, ODS, 4.6 mmID x 250 mm; product of Yamamura Chemical Laboratories,

5

Ltd.]
Detection: 210 nm
Developing solvent: 40% aqueous acetonitrile
Flow rate: 1 ml/min
Retention time (RT): 8.8 min

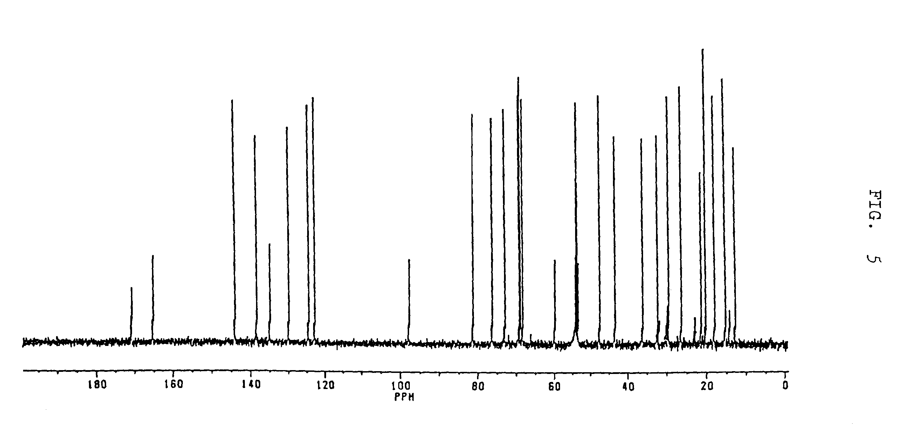

(9) $^{13}$C nuclear magnetic resonance spectrum
(100 MHz, CD$_2$Cl$_2$)
Chart shown in Fig. 5

$\delta_c$: 170.7(s), 165.2(s), 144.0(d), 138.4(d), 134.9(s), 129.9(d), 124.6(d), 122.8(d), 97.8(s), 81.3(d), 76.3(d), 73.0 (d), 69.3(d), 69.0(d), 68.3(d), 59.9(s), 47.5(d), 43.5(t), 36.2(t), 32.5(t), 29.6(d), 26.5(q), 21.4(q), 20.2(q), 18.0 (q), 15.3(q), 12.8(q).

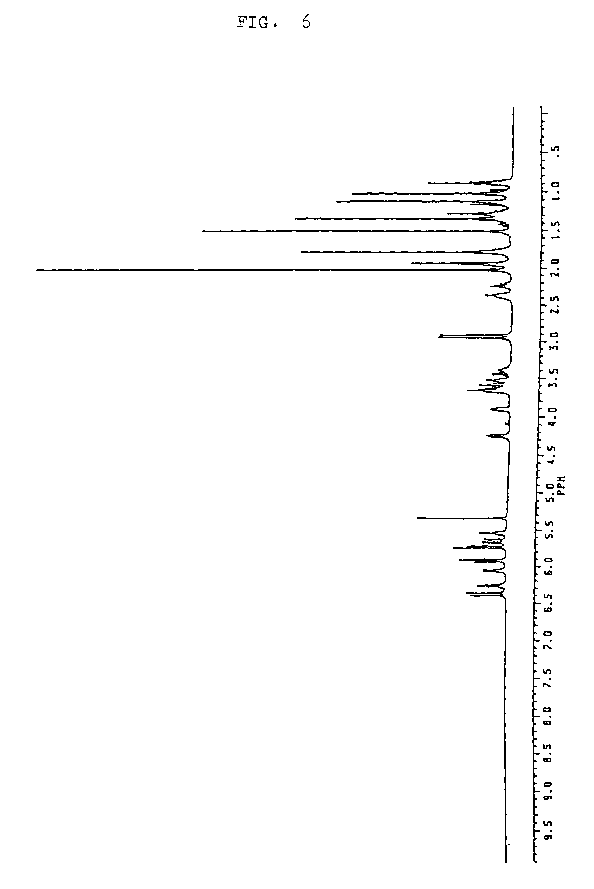

(10) $^{1}$H nuclear magnetic resonance spectrum
(400 MHz, CD$_2$Cl$_2$)
Chart shown in Fig. 6
The significant signals were as follows:

$\delta_H$: 6.37(1H, d, J=16Hz), 6.25(1H, m), 6.04(1H, d, J=9Hz, exchangeable), 5.90(1H, dd, J=11.5 and 8Hz), 5.72 (1H, dd, J=11.5 and 1Hz), 5.64(1H, dd, J=16 and 7Hz), 5.52(1H, m), 5.33(1H, s, exchangeable), 4.24(1H, m), 3.89(1H, m), 3.68-3.49(4H, m), 2.94(1H, d, J=5Hz), 2.90(1H, d, J=5Hz), 2.35(1H, m). 2.23(1H, m), 2.01 (3H, s), 1.92(2H, m), 1.78(3H, br s), 1.75(1H, m), 1.50(3H, s), 1.34(3H, d, J=6.5Hz), 1.11(3H, d, J=6.5Hz), 1.01(3H, d, J=7.5Hz).

(11) Acidity/basicity classification of substance
Neutral substance
(12) Ultraviolet absorption spectrum
$\lambda_{max}^{acetonitrile}$ nm($\in$): 235 (29,000)
(13) Infrared absorption spectrum
$\nu_{max}^{KBr}$ 3400, 2980, 2930, 1735, 1665, 1630, 1520, 1370, 1250, 1115, 1050, 970 cm$^{-1}$

The WB2663 substances according to the present invention are produced by, for example, bacterial strain No. 2663 which the present inventors newly separated from soil samples collected in Mie Prefecture.

Research into the taxonomy of the bacterium of interest was mainly conducted according to the method described in Bergey's Manual of Systematic Bacteriology (Volume 1). The bacteriological properties of the bacteria of interest are given below.

(1) Morphological characteristics

The bacteria of interest were grown on a nutrient agar culture medium at 30°C for 24 hours, after which the morphology thereof was observed under a light microscope. The results are listed in Table 1 below.

Table 1

| Morphological characteristics of strain No. 2663 | |
|---|---|
| Gram stain | negative |
| Color tone of colony | grayish yellow |
| Cell morphology | bacillus |
| Cell size | 0.8-1.0 x 1.5-3.0 μm |
| Mobility | positive |
| Sporulation | negative |

In other words, the bacterium of interest is gram-negative, mobile bacilli. The cells are 0.8-1.0 x 1.5-3.0 μm in size. The bacterium of interest formed a pink soluble pigment on a glucose-peptone agar medium.

(2) Physiological characteristics

The physiological characteristics of the bacterium of interest are listed in Table 2 below.

## Table 2   Physiological properties of strain No. 2663 (1)

| | |
|---|---|
| Growth temperature range | 13–35°C |
| Growth in air | positive |
| Growth on MacConkey's agar medium | positive |
| Catalase | positive |
| Oxidase | positive |
| O-F test | oxidation |
| Utilization of citric acid | positive |
| Reduction of nitrate | negative |
| Indole production | negative |
| $H_2S$ production (SIM) | negative |
| Esculin hydrolysis | negative |
| Starch hydrolysis | negative |
| ONPG test | negative |
| DNase | negative |
| Tween 80 hydrolysis | positive |
| Gelatin hydrolysis | positive |
| Caseine hydrolysis | positive |
| Lysine decarboxylase | positive (weak) |
| Arginine dihydrolase | negative |
| Ornithine decarboxylase | negative |

Table 2    Physiological properties of strain No. 2663 (2)

Acid production from sugars

| | |
|---|---|
| D-glucose | positive |
| D-xylose | negative |
| D-fructose | positive |
| D-mannitol | positive |
| Maltose | negative |
| Sucrose | positive |
| Lactose | positive |
| Salicin | negative |

Table 2    Physiological properties of strain No. 2663 (3)

---

Utilization of sugars and organic acids

| | |
|---|---|
| D-glucose | positive |
| D-arabinose | negative |
| D-mannose | positive |
| D-mannitol | positive |
| N-acetyl-D-glucosamine | positive |
| Maltose | negative |
| Gluconic acid | positive |
| Capric acid | positive |
| Adipic acid | positive |
| Malic acid | positive |
| Citric acid | positive |
| Phenyl acetate | positive |

---

As is clear from Table 2, the temperature range for growth of the bacterium of interest was from 13°C to 35°C. The bacterium was positive for oxidase and catalase, and was oxidative in the O-F test. The bacterium of interest was positive for the decomposition of gelatin, caseine and Tween 80. It was negative for the decomposition of starch, but positive for lysine carboxylase. It produced acids from D-glucose, D-fructose, D-mannitol, sucrose and lactose. It utilized D-glucose, D-mannose and D-mannitol, but did not utilize L-arabinose and maltose.

(3) Identification

As a result of examining the above mentioned characteristics against Bergey's Manual of Systematic Bacteriology (Volume 1), strain No. 2663 was assumed to belong to Pseudomonas. The bacterial strain of interest was thus identified as Pseudomonas sp. No. 2663.

Strain No. 2663 has been deposited at National Institute of Bioscience and Human-Technology, Agency of Industrial Science and Technology, Ministry of International Trade and Industry in Japan (Deposit No.: FERM BP-3421, Deposit date: May 21, 1991).

It should be understood that the production of the present substances is not limited only to the use of the specific microorganism which are described in the present specification and provided solely for explanation. The present invention encompasses the use of all mutants capable of producing the substances of interest, including artificially mutated strains obtained from the listed microorganism using methods of mutagenesis such as X-ray irradiation, ultraviolet irradiation, N-methyl-N'-nitro-N-nitrosoguanidine, 2-aminopurine, etc., as well as naturally mutated strains.

The substances according to the present invention may be produced by inoculating said substance-producing bacterium belonging to the genus Pseudomonas (e.g. Pseudonomas sp. No. 2663) into a nutrient culture medium containing carbon and nitrogen sources which can be utilized by the bacterium, and culturing it under aerobic conditions (e.g. shake culturing, aeration culturing while stirring, etc.).

The carbon sources preferred for use include glucose, sucrose, starch, modified starch, fructose, glycerin and other carbohydrates.

The nitrogen sources preferred for use include oatmeal, yeast extract, peptone, gluten meal, cottonseed flour, cottonseed oil, soybean flour, corn steep liquor, dried yeast, wheat germ, peanut flour, chicken bone and meal meat, etc., but inorganic and organic nitrogen sources such as ammonium salts (e.g. ammonium nitrate, ammonium sulfate, ammonium phosphate, etc.), urea, amino acids, etc. may be used with advantages.

These carbon sources and nitrogen sources may be used together with advantages, and there is no particular need to use them in their pure form. This is because impure forms sometimes contain growth factors and trace elements, which can be advantageous.

If necessary, inorganic salts such as, for example, the following may be added to the culture medium: sodium carbonate, potassium carbonate, sodium phosphate, potassium phosphate, sodium chloride, potassium chloride, sodium iodide, potassium iodide, magnesium salts, copper salts, cobalt salts, etc.

Particularly, if the medium is strongly effervescent, liquid paraffin, animal oils, vegetable oils, mineral oils, silicon, etc. may be added when necessary.

For industrial production of the desired substance in large amounts, aeration culturing while stirring is preferred, as in the case of other fermentates. For production in small amounts, shake culturing using a flask is suitable.

In addition, if culturing is effected in a large tank, it is preferable first to inoculate seed producing cells in a relatively small amount of the medium for cultivation, and then transfer the culture to a large production tank for production culturing, in order to prevent delayed growth of the cells during the process for the production of the substance of interest. In such cases, the respective compositions of both the medium used for pre-culturing and the medium used for production culturing may be identical or modified if necessary.

The culturing is preferably effected under aerated conditions while stirring, and known methods, for example, stirring with a propeller or another device, rotation or shaking of a fermenter, pump processing, blowing with air, etc., are suitable for use. The air used for aeration is sterilized.

The culturing temperature may be appropriately varied within a range in which the substance-producing bacteria can can produce the substance, but cultureing is normally effect at 1-40°C, and preferably 14-36°C. The culturing time differs depending on the conditions and the amount of culturing to be done, but is normally from about 1 day to 1 week.

After completion of fermentation, the desired substance of interest is collected from the culture. That is, the cells are extracted directly with water and/or an organic solvent, or the cells are crushed either mechanically or by a known method such as using ultrasonic waves, etc. after which they are extracted using water and/or an organic solvent, and then collected and purified according to a conventional method. In the case of a culture solution, the substance may be directly collected and purified using a conventional method.

The methods for collection and purification include conventional methods such as solvent extraction using, for example, water, an organic solvent or mixed solvents thereof; chromatography; recrystallization from a single solvent or a solvent mixed solvent, etc., and these may be appropriately employed either alone or in combination.

The isolation and purification of the substance of interest is effected by the appropriate employment of the known methods mentioned above, but each of the 3 effective components may be isolated and purified in the following manner, for example. First, the organic solvent (e.g. ethyl acetate) extract from the culture is passed through a silica gel column, a developing solvent is charged, further column treatment is effected if necessary, and the mixture is subjected to treatment with various developing solvents to obtain fractions rich in WB2663A substance, fractions rich in WB2663B substance and fractions rich in WB2663C substance, respectively. These fractions are then further purified. For example, each of the fractions is extracted with a single or mixed solvent, and the extracts are evaporated or distilled for concentration. The concentrated residues are subjected to chromatography or recrystallization, and lyophilized if necessary, to obtain the further purified components.

A medicinal composition according to the present invention is prepared into a solid, semi-solid or liquid form as a preparation for oral administration or parenteral administration such as an external application, etc., by using 1, 2 or more of the substances of interest and/or their salts as the effective components, and adding a commonly used inorganic or organic carrier thereto.

The preparations for oral administration include tablets, pills, granules, soft/hard capsules, powders, fine granules, dusting powders, emulsions, suspensions, syrups, pellets, elixirs, etc. The preparations for parenteral administration include injections, drips, infusions, pastes, lotions, tonics, sprays, suspensions, oils, emulsions, suppositories, etc. The preparation of an effective component according to the present invention may be done following a conventional procedure, with the appropriate use of a surfactant, excipient, coloring agent, perfume, preservative, stabilizer, buffer, suspending agent, isotonizing agent or other conventionally used auxiliary.

The dosage of a medicinal composition according to the present invention differs depending on its kind, the type of disease for treatment or prevention, the method of administration, the age and symptoms of the patient, and the length of the treatment period. However, the administration of the effective components (substances of interest) per day for an adult is in the range of 0.01-1000 mg/kg, and preferably 0.1-100 mg/kg in the case of intravenous injection; 0.01-1000 mg/kg, and preferably 0.1-100 mg/kg in the case of intramuscular injection; and 0.5-2000 mg/kg, and preferably 1-1000 mg/kg in the case of oral administration.

A more detailed explanation of the present invention is provided below, with reference to the examples.

<u>Example 1</u>

(1) Culturing of strain No. 2663

A medium composed of 1% polypeptone (product of Nihon Seiyaku, Ltd.), 0.5% yeast extract (product of Nihon Seiyaku, Ltd.) and 0.5% NaCl was poured in portions of 160 ml each into 500 ml Erlenmeyer flasks, and sterilized at 120°C for 30 minutes. A slant culture of <u>Pseudomonas</u> sp. No. 2663 (FERM BP-3421) was transplanted thereto one loopful, and culturing was effected at 30°C for 24 hours in a rotary shaker (220 rotations/min), to prepare seed culture. Separately, 20 l of a medium at pH 7.0 containing 1% modified starch, 1% glycerin, 0.5% glucose, 1% defatted soybean meal, 0.5% corn steep liquor, 0.2% $(NH_4)_2SO_4$, 0.006% $MgSO_4 \cdot 7H_2O$, 0.2% $CaCO_3$, 0.05% adecanol (LG-109, Asahi Denka Kogyo K.K.) and 0.05% silicon (KM-70, Shin-Etsu Chemical Co., Ltd.)was poured into each of 30 liter jar fermenters and sterilized at 120°C for 30 minutes, and then 480 ml of the above-mentioned seed culture was added thereto and cultured at 25°C for 2 days, at a aeration volume of 20 l/min, an internal pressure of 1 kg/cm$^2$, and 200 rotations/min.

(2) Isolation/purification of the substances produced by strain No. 2663

After culturing was completed, 400 l of the culture gathered from twenty 30-liter jar fermenters was adjusted to pH 7.0, extracted twice with 1.5-fold volume (600 l) of ethyl acetate, and the extracts were combined for concentration under reduced pressure. To the concentrate was added anhydrous sodium sulfate for dehydration, after which 1.7 l of Silica Gel 60 (70-230 mesh, product of E. Merck Co.) was added thereto, follwed by drying under reduced pressure. The resulting powder was placed on a 4 liter silicagel column (same as above) which had been prefilled with n-hexane, and chromatography was effected. That is, successive elutions were made with 17 l of n-hexane, 17 l of n-hexane: ethyl acetate (3:1), 17 l of n-hexane:ethyl acetate (1:1), 17 l of ethyl acetate and 17 l of ethyl acetate:acetone (1:1), upon which WB2663A substance was eluted in the n-hexane:ethyl acetate (1:1) fraction, and WB2663B and WB2663C substances were eluted in the ethyl acetate fraction.

First, the method of purification of WB2663A substance will be described below. The n-hexane:ethyl acetate (1:1) fraction was concentrated under reduced pressure, and the resulting oily substance (21 g) was combined with 50 ml of silicagel (same as above) and dried under reduced pressure. The resulting powder was placed on a 450 ml silicagel column (same as above) which had been pre-filled with n-hexane, and chromatography was effected. That is, successive elutions were made with 2.5 l of n-hexane, 2.5 l of n-hexane:acetone (8:1), 2.5 l of n-hexane:acetone (6:1), 2.5 l of n-hexane:acetone (4:1), 2.5 l of n-hexane:acetone (2:1) and 2.5 l of n-hexane:acetone (1:1), upon which activity was observed in the n-hexane:acetone (4:1) and (2:1) fractions. The active fractions were combined and concentrated under reduced pressure, the resulting oily substance (8 g) was dissolved in 700 ml of a 10% aqueous solution of acetonitrile, and placed in a 200 ml ODS gel (YMC ODS-AM 120-S50, product of Yamamura Kagaku Co.) column which had been pre-filled with water for reverse phase chromatography. Upon successive elutions of the column with 600 ml each of 10%, 20%, 30% and 40% aqueous solutions of acetonitrile, activity was observed in the fraction of the 40% aqueous solution of acetonitrile. The active fraction was concentrated under reduced pressure and the acetonitrile removed, after which extraction was made twice with equal volumes of ethyl acetate and the extracts were combined and concentrated and dried to solidity. The concentrated residue was dissolved in a small amount of dichloromethane, and n-hexane was added thereto to obtain 512 mg of colorless needle-shaped crystals of WB2663A substance.

Next, an explanation will be given regarding the method of purification of WB2663B substance. The previously mentioned ethyl acetate fraction was concentrated under reduced pressure, and the resulting oily substance (62.9 g) was dissolved in 1.1 l of n-hexane:chloroform (4:7), and placed in 1.2 l of silicagel (same as above) which had been pre-filled with n-hexane:chloroform (1:1). Upon successive elutions of the column with 3.6 l each of chloroform:acetone mixtures (1:0, 100:1, 50:1, 25:1 and 10:1), activity was observed in the chloroform:acetone (100:1) and (50:1) fractions. The WB2663C substance, described later, was eluted in the chloroform:acetone (25:1) fraction. The active fractions were combined and concentrated under reduced pressure, and the resulting oily substance (7.2 g) was dissolved in 2 l of a 20% aqueous solution of acetonitrile and placed in a 200 ml pre-filled ODS gel (same as above) column which had been prepacked with a 20% aqueous solution of acetonitrile for reverse phase chromatography. Upon successive elutions of the column with 600 ml each of 25% and 27% aqueous solutions of acetonitrile, activity was observed in the fraction of the 27% aqueous solution of acetonitrile. The active fraction was concentrated under reduced pressure and the acetonitrile removed therefrom, after which extraction was made twice with equal volumes of ethyl acetate and the extracts were combined and concentrated under reduced pressure to obtain 819 mg of white powder of WB2663B substance.

Next, an explanation will be given regarding the method of purification of WB2663C substance. The previously mentioned chloroform:acetone (25:1) fraction was concentrated under reduced pressure, and the resulting oily substance was dissolved in 300 ml of chloroform:n-hexane (1:1), and placed in a 100 ml silica gel (same as above) column which had been pre-filled with n-hexane:acetone (10:1). Upon elution of the column with 300 ml each of n-hexane: acetone mixtures (5:1, 4:1, 7:2, 3:1, 2:1, 1:1 and 0:1), activity was observed in the n-hexane:acetone (1:1) and acetone fractions. Both of the active fractions were combined and concentrated under reduced pressure, and the resulting oily substance was dissolved in 125 ml of a 20% aqueous solution of acetonitrile and placed in a 200 ml YMC gel (same as above) column which had been pre-filled with a 10% aqueous solution of acetonitrile. Upon elution of the column with 500 ml each of 24% and 26% aqueous solutions of acetonitrile, activity was observed in the fraction of the 26% aqueous solution of acetonitrile. The active fraction was concentrated under reduced pressure and the acetonitrile removed therefrom, after which extraction was made twice with equal volumes of ethyl acetate, the extracts were combined and concentrated to dryness under reduced pressure, the residue was dissolved in a small amount of dichloromethane, and then n-hexane was added thereto to obtain 70 mg of white powder of WB2663C substance.

Example 2

(In vitro human tumor cell growth inhibition by the substance of interest)

As described below, each of the substances of interest were investigated as to their cell toxicities against A549 human pulmonary adenocarcinoma, MCF-7 human mammary adenocarcinoma, SW480 human colon adenocarcinoma, HCT116 human colon adenocarcinoma, P388 mouse leukemia cells and mouse bone marrow (BM) cells. Culturing was initiated at cell concentrations of $4 \times 10^3$/well of the human solid adenocarcinoma cells, $1 \times 10^4$/well of the mouse leukemia cells, and $1 \times 10^5$/well of the mouse bone marrow cells, and the cell toxicities were measured on the 4th day. More specifically, the cytoxicity test was conducted in a 96-well microtitre plate, each well containing the tumor cells at the cell concentrations mentioned above in 100 µl of Dulbecco's minimum essential medium to which had been added 10% fetal calf serum, penicillin (50 units/ml) and streptomycin (50 µg/ml). The cells were incubated at 37°C for 4 days, and a colorimetric MTT (3-(4,5-dimethylthiazole-2-il)-2,5-diphenyltetrazolium bromide, product of Sigma Co.) assay was made according to the method described by Mosmann in J. Immunol. Methods, 65, 55-63, 1983.

First, MTT was dissolved in a phosphate buffer solution (PBS) to a concentration of 5 mg/ml, and the solution was filtered and sterilized to remove the small amount of insoluble residue. After completion of culturing of the tumor cells, the MTT solution was added to all of the assay wells (10 µl per 100 µl of medium), and the plate was further incubated at 37°C for 4 hours. Acidic isopropanol (100 µl of 0.04 N HCl in isopropanol) was added to all of the wells, and mixing was effected to dissolve the dark blue crystals. After all of the crystals dissolved, the plate was subjected to a dual wavelength photometer (Model MIP-22: Corona Denki Co., Inc., Katsuta-shi, Japan) at 550 nm and a reference wavelength of 660 nm, and read. The object compounds according to the present invention were dissolved in methanol and diluted with Dulbecco's minimum essential medium, and added to the cultures to a final concentration of 1 µg/ml or less. The results are shown in Table 3.

Table 3

| In vitro human tumor cell growth inhibition by the substance of interest | | | |
|---|---|---|---|
| Cell line | WB2663A substance | IC$_{50}$ (ng/ml) WB2663B substance | WB2663C substance |
| MCF-7 | 0.46 | 0.91 | 0.59 |
| A549 | 0.35 | 0.66 | 0.44 |
| HCT116 | 0.22 | 0.31 | 0.34 |
| SW480 | 0.40 | 0.51 | 0.53 |
| P388 | 0.82 | 1.69 | 0.48 |
| BM | 2.03 | 5.01 | 1.91 |
| MCF-7: human mammary adenocarcinoma<br>A549: human pulmonary adenocarcinoma<br>HCT116: human colon adenocarcinoma<br>SW480: human colon adenocarcinoma<br>P388: mouse leukemia cells<br>BM: mouse bone marrow cells | | | |

As is clear from the results in Table 3, all 3 components of the substance of interest exhibited a strong cytoxicity against all of the human solid cancer, as well as cytoxicity, though weaker, against mouse leukemia cells (WB2663A substance and WB2663B substance) and mouse bone marrow cells.

Example 3

(Life lengthening test with P388 mouse leukemia cells)

P388 mouse leukemia cells were intraperitoneally transplanted to $BDF_1$ mice (female, 7 weeks old) at $1 \times 10^6$ cells/mouse ($5 \times 10^6$ cells/ml x 0.2 ml/mouse), and from the following day, WB2663A substance, WB2663B substance and WB2663C substance dissolved in 10% HCO-60 [polyoxy ethylated (60 mole) hydrogenated castor oil in saline (product of Nikko Co.)] (pH 7.0) were each intraperitoneally administered once a day for 4 days (d1, d2, d3, d4), at doses of 0.2 ml/mouse. To the control mice, 10% HCO-60 (same as above) was administered. The numbers d1-d4 indicate the data from day 1 to day 4, respectively.

The results are shown in Table 4 below. The antitumor activity was expressed as the number of animals died/ number of animals treated (D/T), the body weight change during the treatment period (d4-d1), the median survival time (MST) of the group, and T/C% (100 x treated group/control group) of the median survival time.

Table 4   Life  lengthening  effect  of  the  substance  of
interest (1)

WB2663A substance

| Dose (mg/kg) | D/T | Body weight change d4-d1 (g) | MST (days) | T/C (g) |
|---|---|---|---|---|
| Control | 0/5 | 0.4 | 10 | 100 |
| 0.1 | 0/5 | 0.1 | 10 | 100 |
| 0.18 | | | | |
| 0.32 | 0/5 | 0.3 | 11 | 110 |
| 1.0 | 0/5 | −0.3 | 16 | 160 |
| 3.2 | 1/5 | −1.7 | 14.5 | 145 |

Table 4   Life  lengthening  effect  of  the  substance  of
interest (2)

WB2663B substance

| Dose (mg/kg) | D/T | Body weight change d4-d1 (g) | MST (days) | T/C (g) |
|---|---|---|---|---|
| Control | 0/5 | 0.4 | 11 | 100 |
| 0.018 | 0/5 | 0 | 13 | 118 |
| 0.032 | 0/5 | −0.4 | 12 | 109 |
| 0.056 | 0/5 | −0.6 | 15 | 136 |
| 0.1 | 0/5 | −1.6 | 16 | 145 |
| 0.18 | 2/5 | −2.0 | 15 | 136 |

Table 4   Life  lengthening  effect  of  the  substance  of
interest (3)

WB2663C substance

| Dose (mg/kg) | D/T | Body weight change d4-d1 (g) | MST (days) | T/C (g) |
|---|---|---|---|---|
| Control | 0/5 | 0.4 | 11 | 100 |
| 0.0032 | 0/5 | 0.4 | 12 | 109 |
| 0.01 | 0/5 | −0.3 | 13 | 118 |
| 0.032 | 0/5 | −1.6 | 14 | 127 |
| 0.056 | | | | |
| 0.1 | 4/5 | −2.8 | 17 | 155 |

As is clear from the results in Table 4, a life lengthening effect was exhibited towards P388 mouse leukemia tumor-bearing mice at 1 mg/kg and 3.2 mg/kg of WB2663A substance, 0.056 mg/kg, 0.1 mg/kg and 0.18 mg/kg of WB2663B substance, and 0.032 mg/kg of WB2663C substance.

Example 4

(Antitumor effect against A549 human pulmonary adenocarcinoma cells)

A549 human pulmonary adenocarcinoma cells which had been subcultured under the skin of nude mice (female, 7 weeks old) were cut into slices 1 mm square and implanted one under the renicapsule of $BDF_1$ mice (female, 7 weeks old). On the following day (first day), the fifth day and the ninth day, WB2663A substance or WB2663B substance which had been dissolved in 10% HCO-60 [polyoxyethlated (60 mole) hydrogenated castor oil in saline (produce of Nikko Co.)] (pH 7.0) was intraperitoneally administered at dose of 0.2 ml/mouse. To the control group, 10% HCO-60 (same as above) was administered. In addition, to promote the survival of the tumors, 32 mg/kg of immunosuppressant agent FK506 ( Fujisawa Pharmaceutical Co., Ltd. ) suspended in physiological saline was subcutaneously administered at dose ( 32 mg/kg ) of 0.2 ml/mouse on the 1st, 2nd, 5th, 7th, 9th and 12th days. Groups of 8 mice each were used for the experiment. At 14 days after implanting, the antitumor activity was measured. The results are shown in Table 5 below (d1-d14 represent the data of the first to the 14th days, respectively). The antitumor activity was expressed as the number of animals died/number of animals treated (D/T), the body weight change during the treatment period (d14-d1), the median tumor volume of each group ($mm^3$), and the tumor volume inhibition rate (%). The tumor volume was calculated using the equation: Tumor volume ($mm^3$) = (major axis of tumor) x (minor axis of tumor)$^2$/2, while the tumor volume inhibition rate was calculated using the equation: Inhibition (%) = (median tumor volume of control group - median tumor volume of treated group)/median tumor volume of control group x 100.

Table 5        Antitumor effect of WB2663A against A549 (1)

| Drug | Dose (mg/kg) | D/T | Body weight change (g) [d14-d1] | tumor volume (mm³) | inhibition (%) |
|------|------|------|------|------|------|
| WB2663A | Control | 0/8 | 0.53 | 14.87±3.75 | — |
|  | 0.56 | 0/7 | −0.39 | 14.17±2.63 | 5.2 |
|  | 1.0 | 0/7 | −1.17 | 15.87±2.31 | −6.7 |
|  | 1.8 | 0/8 | −0.71 | 12.89±1.86 | 13.3 |
|  | 3.2 | 2/8 | −1.98 | 5.79±1.15 | 61.0 |
|  | 5.6 | 8/8 | — | — | — |

Table 5        Antitumor effect of WB2663B against A549 (2)

| Drug | Dose (mg/kg) | D/T | Body weight change (g) [d14-d1] | tumor volume (mm³) | inhibition (%) |
|------|------|------|------|------|------|
| WB2663B | Control | 0/8 | 1.6 | 29.16±5.08 | — |
|  | 0.056 | 0/7 | 1.5 | 18.53±3.65 | 36.4 |
|  | 0.10 | 0/8 | 1.0 | 11.36±1.36** | 61.1 |
|  | 0.18 | 0/8 | 0.2 | 10.74±2.34** | 63.2 |
|  | 0.32 | 1/8 | −0.5 | 6.63±1.02** | 77.3 |
|  | 0.56 | 2/8 | −1.4 | 4.85±1.46** | 83.4 |
|  | 1.00 | 5/7 | −2.3 | 10.58±5.72 | 63.7 |

Example 5

(Life lengthening test with Meth A. mouse fibrosarcoma cells)

Mouse fibrosarcoma Meth A cells were intradermally transplanted to Balb/c mice (female, 7 weeks old) at $1 \times 10^5$ cells/mouse. WB2663B substance was diluted to various medicinal concentrations using physiological saline containing 10% HCO-60, and was intravenously administered 3 times on the 8th, 11th and 14th days (d8, 11 and 14) after transplantation. To the control mice, 10% HCO-60 was administered. Groups of 10 mice each were used for the experiment.

The antitumor activity was measured on the 17th day after transplantation, and the results are shown in Table 6 below.

$$\text{Inhibition\%} = (1 - \frac{Tn/To}{Cn/Co}) \times 100$$

The antitumor activity was expressed as growth inhibition rate relative to the tumor weight:

Tn:  tumor weight of treated group
Cn:  tumor weight of control group
To:  tumor weight of treated group at time of first administration of drug
Co:  tumor weight of control group at time of first administration of drug

The relative tumor weight was then calculated by:

$$\text{Tumor weight} = \frac{a \times b^2}{2}$$

a:  major axis of tumor
b:  minor axis of tumor

Table 6

| Antitumor effect of WB2663B against Meth A | | | |
|---|---|---|---|
| Drug | Dose (mg/kg) | Body weight change (g) [day17-8] | Tumor weight(mg) Mean±S.E.(Inhibition%) (day 17) |
| WB2663B | Control | 1.8 | 1421± 86 |
| | 0.1 | 0.6 | 1076±123**(24) |
| | 0.18 | 0.3 | 683±104**(52) |
| | 0.32 | 0.6 | 737±120**(48) |
| | 0.56 | -0.1 | 587± 94**(59) |
| | 1.0 | -0.8 | 156± 58**(89) |

Example 6

(Acute toxicity of the substance of interest)

The acute toxicity test was conducted using $BDF_1$ mice (female, 7 weeks old) with a single intraperitoneal administration. The results were $LD_{50}$ values of 10-5.6 mg/kg for WB2663A substance, 3.2 mg/kg for WB2663B substance, and 0.32-0.1 mg/kg for WB2663C substance.

References to the deposited microorganism under Rule 13-2

1. Pseudonomas sp. No. 2663

    a) Name and address of the depository authority in which said microorganism is deposited:
       Name: National Institute of Bioscience and Human-Technology, Agency of Industrial Science and Technology, Ministry of International Trade and Industry
       Address: 1-3, Higashi 1 chome, Tsukuba-shi, Ibaraki-ken, 305, Japan
    b) Date of deposit at the depository authority shown in a) May 21, 1991
    c) Deposit No. given by the depository authority shown in a) FERM BP-3421

**Claims**

1.  WB2663A substance characterized by possessing the properties indicated below:

    (A) Melting point
         102-104°C
    (B) Specific rotation
         $[\alpha]_D^{23}$: -36° (c=0.5, $CH_2Cl_2$)
    (C) Elemental analysis

| Calculated for $C_{27}H_{42}ClNO_8$ (%) : | | | |
|---|---|---|---|
| C, 59.60; | H, 7.78; | N, 2.57; | Cl, 6.52 |
| Found (%) : | | | |
| C, 60.11; | H, 8.10; | N, 2.44; | Cl, 6.86 |

(D) Infrared absorption spectrum

$\nu_{max}^{KBr}$ 3400, 2980, 2940, 1735, 1665, 1630, 1530, 1370, 1240, 1055 cm$^{-1}$

**2.** WB2663B substance characterized by possessing the properties indicated below:

(A) Melting point

65-70°C

(B) Specific rotation

$[\alpha]_D^{23}$: -12° (c=0.5, $CH_2Cl_2$)

(C) Molecular formula and molecular weight

HRFAB-MS m/z 508.2929

$C_{27}H_{41}NO_8$ + H : 508.2910

(D) Ultraviolet absorption spectrum

235 nm ($\in$=30,500, in acetonitrile)

**3.** WB2663C substance characterized by possessing the properties indicated below:

(A) Melting point

86-91°C

(B) Specific rotation

$[\alpha]_D^{23}$: -9° (c=0.5, $CH_2Cl_2$)

(C) Molecular formula and molecular weight

HRFAB-MS m/z 524.2849

$C_{27}H_{41}NO_9$ + H : 524.2860

(D) Ultraviolet absorption spectrum

235 nm ($\in$=29,000, in acetonitrile)

**4.** A method for the production of WB2663 substances, characterized by culturing WB2663 substance-producing bacteria belonging to the genus <u>Pseudomonas</u> to produce WB2663 substances, and collecting them.

**5.** An antitumor agent containing, as an effective component, WB2663A substance, WB2663B substance and/or WB2663C substance.

**Patentansprüche**

**1.** WB2663A-Substanz, dadurch **gekennzeichnet,** daß sie folgende Eigenschaften besitzt:

(A) Schmelzpunkt:

102-104°C

(B) Spezifische Rotation:

$[\alpha]_D^{23}$: -36° (c=0,5, $CH_2Cl_2$)

(C) Elementaranalyse:

| berechnet für $C_{27}H_{42}ClNO_8$ (%): | | | |
|---|---|---|---|
| C: 59.60; | H: 7.78; | N: 2.57; | Cl: 6.52 |
| gefunden (%): | | | |
| C: 60.11; | H: 8.10; | N: 2.44; | Cl: 6.86 |

(D) Infrarot-Absorptionsspektrum:
$\nu_{max}^{KBr}$ 3400, 2980, 2940, 1735, 1665, 1630, 1530, 1370, 1240, 1055 cm$^{-1}$.

2. WB2663B-Substanz, dadurch **gekennzeichnet,** daß sie folgende Eigenschaften besitzt:

(A) Schmelzpunkt:
65-70°C
(B) Spezifische Rotation:
$[\alpha]_D^{23}$: -12° (c=0,5, CH$_2$Cl$_2$)
(C) Molekülformel und Molekulargewicht:
HRFAB-MS m/z 508,2929
C$_{27}$H$_{41}$ClNO$_8$ + H: 508,2910
(D) Ultraviolett-Absorptionsspektrum:
235 nm ($\varepsilon$=30.500, in Acetonitril).

3. WB2663C-Substanz, dadurch **gekennzeichnet,** daß sie folgende Eigenschaften besitzt:

(A) Schmelzpunkt:
86-91°C
(B) Spezifische Rotation:
$[\alpha]_D^{23}$: -9° (c=0,5, CH$_2$Cl$_2$)
(C) Molekülformel und Molekulargewicht:
HRFAB-MS m/z 524,2849
C$_{27}$H$_{41}$ClNO$_9$ + H: 524,2860
(D) Ultraviolett-Absorptionsspektrum:
235 nm ($\varepsilon$=29.000, in Acetonitril).

4. Verfahren zur Herstellung von WB2663-Substanzen, **gekennzeichnet** durch Züchten von WB2663-Substanz produzierende Bakterien, die zur Gattung Pseudomonas gehören, zur Herstellung von WB2663-Substanzen und Sammeln derselben.

5. Antitumormittel, das als wirksamen Bestandteil die WB2663A-Substanz, die WB2663B-Substanz und/oder die WB2663C-Substanz enthält.

**Revendications**

1. Substance WB2663A caractérisée en ce qu'elle possède les propriétés indiquées ci-dessous :

(A) Point de fusion :
102-104°C.
(B) Pouvoir rotatoire spécifique :
$[\alpha]_D^{23}$ : -36° (c = 0,5, CH$_2$Cl$_2$).
(C) Composition élémentaire :

| Calculé pour C$_{27}$H$_{42}$ClNO$_8$ (%) : | | | |
|---|---|---|---|
| C:, 59.60; | H, 7.78; | N, 2.57; | Cl, 6.52 |
| Trouvé (%) : | | | |
| C, 60.11; | H, 8.10; | N, 2.44; | Cl, 6.86. |

(D) Spectre d'absorption infra-rouge :
$\nu_{max}^{KBr}$ 3400, 2980, 2940, 1735, 1665, 1630, 1530, 1370, 1240, 1055 cm$^{-1}$.

2. Substance WB2663B caractérisée en ce qu'elle possède les propriétés indiquées ci-dessous :

(A) Point de fusion :
65-70°C.

(B) Pouvoir rotatoire spécifique :

$[\alpha]_D^{23}$ : -12° (c = 0,5, $CH_2Cl_2$).

(C) Formule brute et masse moléculaire :

HRFAB-SM m/z 508,2929.

$C_{27}H_{41}NO_8$ + H : 508 2910).

(D) Spectre d'absorption dans l'ultraviolet :

235 nm ($\varepsilon$=30 500, dans l'acétonitrile).

3. Substance WB2663C caractérisée en ce qu'elle possède les propriétés indiquées ci-dessous :

(A) Point de fusion :

86-91°C.

(B) Pouvoir rotatoire spécifique :

$[\alpha]_D^{23}$ : -9° (c = 0,5, $CH_2Cl_2$).

(C) Formule brute et masse moléculaire :

HRFAB-SM m/z 524,2849.

$C_{27}H_{41}NO_9$ + H : 524 2860).

(D) Spectre d'absorption dans l'ultraviolet :

235 nm ($\varepsilon$=29 000, dans l'acétonitrile).

4. Procédé de préparation des substances WB2663, caractérisé en ce qu'on cultive des bactéries produisant la substance WB2663 appartenant au genre Pseudomonas pour produire les substances WB2663 et en ce qu'on les recueille.

5. Agent antitumoral contenant comme constituant actif la substance WB2663A, la substance WB2663B et/ou la substance WB2663C.

FIG. 1

FIG. 2

FIG. 3

FIG. 4

FIG. 5

FIG. 6